# EUROPEAN PATENT APPLICATION

(11) **EP 4 545 957 A1**
(43) Date of publication of application: **30.04.2025**
(21) Application number: 23826812.2
(22) Date of filing: 28.04.2023
(51) Int. Cl.: G01N 27/00, G01N 33/543

(54) **METHOD AND PROGRAM FOR DETECTING AND QUANTIFYING PROTEIN**

(30) Priority: 24.06.2022 JP 2022101993
(71) Applicant: Aipore Inc., Tokyo, 150-8512 (JP)
(72) Inventor: NAONO, Norihiko, Tokyo 150-8512 (JP); SAKAMOTO,Osamu, Tokyo 150-8512 (JP); TAKEI,Hiroyasu, Tokyo 150-8512 (JP)
(74) Representative: Mewburn Ellis LLP
(86) International application number: PCT/JP2023/016944
(87) International publication number: WO 2023/248623

(57) **Abstract**

A method for quantifying an antigen or antibody, the method using a sensor including two chambers separated by a partition wall and communicating with each other through a pore, and an electrode in each of the two chambers; the method including:
preparing a known measurement target sample by mixing:
antibody-modified particles having an antibody that specifically binds to the antigen attached to a particle surface, or antigen-modified particles having an antigen that specifically binds to the antibody attached to a particle surface,
a first electrolyte solution, and
a known sample having a known concentration of the antigen or the antibody,
wherein the antigen or the antibody is contained at a first concentration, and the antibody-modified particles or the antigen-modified particles are contained at a second concentration, respectively;

filling one of two chambers of a first sensor with the known measurement target sample;
filling the other of the two chambers of the first sensor with a second electrolyte solution to electrically connect the two chambers through a pore;
applying a voltage between the electrodes of each of the two chambers and causing an ion current to flow between the electrodes via the pore, and measuring transient change in the ion current as a known pulse waveform group consisting of a plurality of known pulse waveforms measured at a known pulse frequency equal to or higher than a lower limit frequency;
creating a correlation model between a known pulse waveform feature expressing a shape of the known pulse waveform, or a known distribution feature expressing a distribution characteristic of the known pulse waveform feature within the group of known pulse waveforms, and a concentration of the antigen or the antibody in the known sample;
using the correlation model, determining a value at which a change in the concentration of the antigen or the antibody with respect to the known pulse waveform feature or the known distribution feature exceeds a predetermined threshold, and defining the value as an agglutination accelerating concentration; and
estimating a concentration of the antigen or the antibody contained in an unknown sample by using the correlation model and the agglutination accelerating concentration.

## Description

### FIELD OF THE INVENTION

The present invention relates to a method and a program for detecting and quantifying a protein contained in a sample.

### BACKGROUND OF THE INVENTION

Since proteins are the basic components of living organisms, their detection and quantification are fundamental tools in biological research, and are widely used in clinical testing for various diseases.

A widely used method in clinical testing is the so-called immunoassay, which uses the protein to be detected or quantified as an antigen and utilizes the phenomenon in which measurement particles, the surfaces of which are modified with antibodies that specifically bind to the antigen, bind to each other via the antigen and agglutinate. There are various methods for observing such agglutination of antibody-modified particles. For example, turbidimetry, absorbance method, immunochromatography, and the like, which measure changes in light absorption or reflection due to the aggregation phenomenon, or alternatively, ELISA (Enzyme-Linked Immuno Sorbent Assay), CLEIA (Chemiluminescent Enzyme Immunoassay), and CLIA (Chemiluminescent Immunoassay), which label antibodies or antigens with chemiluminescent substances to measure the absorbance or luminescence, are widely used.

Among these, turbidimetry, absorbance, immunochromatography, and the like have simple testing procedures and are low cost, but have low sensitivity and are limited in use to testing test samples with high antigen concentrations. On the other hand, although ELISA, CLEIA and CLIA are generally more sensitive than the above-mentioned methods of observing particle agglutination, they have problems in that the testing procedures are complicated and require expensive equipment and reagents, making it difficult to achieve both high sensitivity and low cost.

In these circumstances, a technique known as the pore electrical resistance method (Patent Literature 1) has been proposed, in which nano-sized particles in an electrolyte are driven by electrophoresis and the transient change in electrical resistance is observed as the particles pass through pores close to the size of the particles, and a technique has been proposed that aims to achieve both high sensitivity and low cost by directly measuring the target protein (Non-Patent Literature 1).

### PRIOR ART

### Patent Literature

[Patent Literature 1] U.S. Patent No. 9,726,636

### Non-Patent Literature

[Non-Patent Literature 1] Yusko, Erik C., et al. "Real-time shape approximation and fingerprinting of single proteins using a nanopore." Nature nanotechnology 12.4 (2017): 360-367.

### SUMMARY OF THE INVENTION

The problem with techniques for observing the agglutination of antibody-modified particles using light, such as turbidimetry, spectrophotometry, and immunochromatography, is that they have low sensitivity. On the other hand, the problems with chemiluminescence-based techniques such as ELISA and CLEIA are the high cost and complicated processing. Conventional techniques have been unable to achieve both high sensitivity and inexpensive, rapid testing.

On the other hand, for example, there remain major challenges to be overcome before a new proposed technology for measuring proteins using electrical resistance in pores can be put to practical use. As described in Non-Patent Literature 1, it is possible in principle to detect and quantify proteins. However, from the viewpoint of application to clinical tests and the like, there are the following three problems.

The first problem is that it is difficult to mass-produce the pore device at low cost. For example, if the specific gravity is 1.2 g/cm³ at 60 kDa, the diameter is about 5.4 nm when it is approximated as a sphere. In order to measure particles of this size using the pore electrical resistance method, the pore diameter must be at most 30 nm to 10 nm. For example, even if one were to mass-produce micropore devices of this size using photolithography technology for semiconductor integrated circuits as in Patent Literature 1, general exposure technology could not be used, and it would be necessary to use an expensive, low-throughput technology such as an electron beam lithography apparatus.

The second problem is that biological samples contain various contaminating particles that pass through the pores, making it difficult to obtain a pore-passing signal for only the target protein.

The third problem is that it is not possible to measure only the proteins that are the target of detection or quantification. A biological sample that needs to be measured for practical applications contains countless types of proteins, and since these pass through the pores nonspecifically during measurement, it is not possible to specifically measure the proteins that are the target of detection or quantification, making practical detection and quantification difficult.

The pore electrical resistance method is capable of directly observing particles on the order of nanometers that cannot be observed by optical methods, and is also a simple means of measurement. Therefore, if the problems associated with application to clinical testing as described above can be overcome, highly sensitive and inexpensive protein detection and quantification can be achieved. In the present invention, unlike Non-Patent Literature 1, the passage of the protein to be detected or quantified through the pore is not directly measured, but rather the protein to be detected or quantified is used as an antigen, and the passage of antibody-modified particles, in which the surface of detection particles is modified with an antibody that specifically binds to the antigen, is measured. The aggregation state of such antibody-modified particles changes depending on the presence and amount of antigen, and by observing this change using the pore electrical resistance method, the antigen protein can be detected and quantified.

Compared with conventional methods for directly measuring proteins, the method of the present invention offers greater room for optimization of the particle size of the antibody-modified particles, the pore size, the concentration of the antibody-modified particles, and the like. For example, the first problem as described above can be avoided by selecting a pore diameter of 300 nm or more, which allows the pore device to be manufactured by general-purpose photolithography techniques. Further, the second problem as described above can be avoided by filtering the biological sample through a fine filter of about 100 kDa to eliminate contaminating particles other than the protein to be measured before measurement. Furthermore, regarding the third problem, the high specificity of the antigen-antibody reaction makes it possible to eliminate the influence of proteins other than those to be detected or quantified.

However, until now, the optimal conditions for such techniques, such as particle size, pore size, antibody-modified particle concentration, antibody-modified particle substrate, and antibody modification method, have remained unknown, and none of the above problems have been resolved. The present invention has been made in light of these circumstances, and provides a means for detecting and quantifying proteins (or antibodies) with high sensitivity, at low cost, quickly, and simply by finding the optimal condition for the concentration of antibody-modified particles (or antigen-modified particles) in a measurement target sample, which is a particularly important condition. That is, the present invention can provide the following aspects.

### [Aspect 1]

A method for quantifying an antigen or antibody, the method using a sensor comprising two chambers separated by a partition wall and communicating with each other through a pore, and an electrode in each of the two chambers; the method comprising:
preparing a known measurement target sample by mixing:
   antibody-modified particles having an antibody that specifically binds to the antigen attached to a particle surface, or antigen-modified particles having an antigen that specifically binds to the antibody attached to a particle surface,
   a first electrolyte solution, and
   a known sample having a known concentration of the antigen or the antibody,
   wherein the antigen or the antibody is contained at a first concentration, and the antibody-modified particles or the antigen-modified particles are contained at a second concentration, respectively;
filling one of two chambers of a first sensor with the known measurement target sample;
filling the other of the two chambers of the first sensor with a second electrolyte solution to electrically connect the two chambers through a pore;
applying a voltage between the electrodes of each of the two chambers and causing an ion current to flow between the electrodes via the pore, and measuring transient change in the ion current as a known pulse waveform group consisting of a plurality of known pulse waveforms measured at a known pulse frequency equal to or higher than a lower limit frequency;
creating a correlation model between a known pulse waveform feature expressing a shape of the known pulse waveform, or a known distribution feature expressing a distribution characteristic of the known pulse waveform feature within the group of known pulse waveforms, and a concentration of the antigen or the antibody in the known sample;
using the correlation model, determining a value at which a change in the concentration of the antigen or the antibody with respect to the known pulse waveform feature or the known distribution feature exceeds a predetermined threshold, and defining the value as an agglutination accelerating concentration; and
estimating a concentration of the antigen or the antibody contained in an unknown sample by using the correlation model and the agglutination accelerating concentration.

### [Aspect 2]

The method according to aspect 1, wherein the step of estimating the concentration of the antigen or the antibody contained in the unknown sample by using the correlation model and the agglutination accelerating concentration comprises:
preparing an unknown measurement target sample by mixing:
   the antibody-modified particles or the antigen-modified particles,
   the first electrolyte solution, and
   the unknown sample,
   wherein the antibody-modified particles or the antigen-modified particles are contained at a second concentration;
filling the unknown measurement target sample into one of the two chambers of a second sensor;
filling the other of the two chambers of the second sensor with the second electrolyte solution to electrically connect the two chambers through a pore;
applying a voltage between the electrodes of each of the two chambers and causing an ion current to flow between the electrodes via the pore, and measuring transient change in the ion current as an unknown pulse waveform group consisting of a plurality of unknown pulse waveforms measured at an unknown pulse frequency equal to or higher than a lower limit frequency; and
estimating a concentration of an antigen or antibody in the unknown sample by comparing an unknown pulse waveform feature that expresses a shape of the unknown pulse waveform, or an unknown distribution feature that expresses a distribution characteristic of the unknown pulse waveform feature within the unknown pulse waveform group, with the correlation model.

### [Aspect 3]

The method according to aspect 1 or 2, wherein the second concentration is set so that the aggregation accelerating concentration is lower than the first concentration.

### [Aspect 4]

The method according to aspect 2 or 3, wherein the lower frequency limit of the unknown pulse rate is 2 pulses per minute.

### [Aspect 5]

The method according to any one of aspects 1 to 4, wherein the aggregation accelerating concentration is determined such that:
a difference between a reference pulse waveform feature at the agglutination accelerating concentration and a reference pulse waveform feature at an antigen concentration or antibody concentration that is 10 times the agglutination accelerating concentration is equal to or greater than twice of
a difference between the reference pulse waveform feature at the agglutination accelerating concentration and the reference pulse waveform feature at an antigen concentration or antibody concentration that is 1/10 of the agglutination accelerating concentration.

### [Aspect 6]

The method according to any one of aspects 1 to 5, wherein the aggregation accelerating concentration is determined such that:
a first difference between a distribution feature at the agglutination accelerating concentration and a reference distribution feature at an antigen concentration or antibody concentration that is 10 times the agglutination accelerating concentration is equal to or greater than twice of
a second difference between the distribution feature at the agglutination accelerating concentration and the reference distribution feature at an antigen concentration or antibody concentration that is 1/10 of the agglutination accelerating concentration.

[Aspect 7]

The method according to any one of aspects 2 to 6, wherein the first electrolyte and the second electrolyte have different compositions.

### [Aspect 8]

The method according to any one of aspects 2 to 7, wherein the first sensor and the second sensor are the same sensor in reality.

### [Aspect 9]

A program, comprising computer-readable instructions configured to be executed by a processor in a computer in which the processor is configured to be connected to a sensor via a network, the sensor comprising two chambers separated by a partition wall and communicating with each other through a pore, and an electrode in each of the two chambers; the computer readable instructions configured to execute the processor to perform the steps of:
a step of preparing a known measurement target sample by mixing:
   antibody-modified particles having an antibody that specifically binds to an antigen attached to a particle surface, or antigen-modified particles having an antigen that specifically binds to an antibody attached to a particle surface,
   a first electrolyte solution, and
   a known sample having a known concentration of the antigen or the antibody,
   wherein the antigen or the antibody is contained at a first concentration, and the antibody-modified particles or the antigen-modified particles are contained at a second concentration, respectively;
a step of filling one of two chambers of a first sensor with the known measurement target sample, by the processor;
a step of filling the other of the two chambers of the first sensor with a second electrolyte solution to electrically connect the two chambers through a pore, by the processor;
a step of applying a voltage between the electrodes of each of the two chambers and causing an ion current to flow between the electrodes via the pore, and measuring transient change in the ion current as a known pulse waveform group consisting of a plurality of known pulse waveforms measured at a known pulse frequency equal to or higher than a lower limit frequency, by the processor;
a step of creating a correlation model between a known pulse waveform feature expressing a shape of the known pulse waveform, or a known distribution feature expressing a distribution characteristic of the known pulse waveform feature within the group of known pulse waveforms, and a concentration of the antigen or the antibody in the known sample, by the processor;
a step of using the correlation model, determining a value at which a change in the concentration of the antigen or the antibody with respect to the known pulse waveform feature or the known distribution feature exceeds a predetermined threshold, and defining the value as an agglutination accelerating concentration, by the processor; and
a step of estimating a concentration of the antigen or the antibody contained in an unknown sample by using the correlation model and the agglutination accelerating concentration, by the processor.

According to the present invention, protein (or antibody) detection and quantification can be performed with high sensitivity, low cost, rapidity, and simplicity.

### BRIEF DESCRIPTION OF THE DRAWINGS

FIG. 1A shows an example of the device structure of a sensor used in the present invention.
FIG. 1B shows an electron microscope image of a pore used to measure a pulse waveform.
FIG. 2 shows an example of a transient change in ion current.
FIG. 3A shows a chart explaining a method of measuring a known sample in which the presence or absence and concentration of a target antigen are known, and modeling the correlation between the presence or absence and concentration of the target antigen and the shape or distribution characteristics of a pulse signal obtained by the method of the present invention.
FIG. 3B shows a chart explaining a method of measuring an unknown sample in which the presence or absence and concentration of a target antigen are unknown, and comparing the results with the model to detect and quantify a target antigen in the unknown sample.
FIG. 4 shows a schematic representation of a chamber filled with a measurement target sample.
FIG. 5 shows the distribution of pulse waveform characteristics, which are the results of measuring a known sample for the presence or absence and concentration of PSA, an example of a target antigen.
FIG. 6 shows the target antigen concentrations with known pulse frequencies for two types of target antigen.
FIG. 7A shows an example of a feature value expressing the shape of one pulse waveform.
FIG. 7B shows an example of a feature that expresses the characteristics of the histogram shape for the frequency distribution of one feature within a group of pulse waveforms obtained from one measurement.
FIG. 8A is plot of the number of pulses per minute and the ratio of pulses equal to or above the lower peak current limit against the target antigen concentration of influenza N protein.
FIG. 9 is a diagram summarizing the relationship between antibody-modified particle concentration and agglutination-accelerating antigen concentration in the example of FIG. 8.

### DETAILED DESCRIPTION OF THE INVENTION

In an embodiment of the present invention, the detection and quantification of antigens in a biological sample can be performed, and the detection and quantification of antibodies in a biological sample can be performed. In the former case, antibody-modified particles are used whose surfaces are modified with antibodies that specifically bind to the antigen to be detected or quantified (hereinafter referred to as "target antigen"). In the latter case, antigen-modified particles are used whose surfaces are modified with an antigen that specifically binds to the antibody to be detected or quantified (hereinafter referred to as the "target antibody"). When the antibody-modified particles are used to detect or quantify a target antigen, and when the antigen-modified particles are used to detect or quantify a target antibody, the procedure and principle of the present invention are the same. In order to avoid unnecessarily lengthy description, the following will explain the former example, that is, the detection or quantification of a target antigen using antibody-modified particles.

In addition, in another embodiment of the present invention, an antibody in a biological sample can be detected and quantified. That is, this embodiment can be understood by replacing antigen with antibody and antibody with antigen in the above and following descriptions. Although the explanation will not be repeated below, it should be noted that each description in this specification can be interpreted as described above as appropriate to the extent that there is no contradiction.

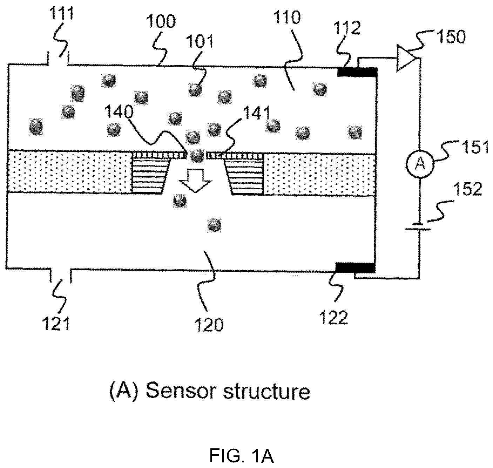

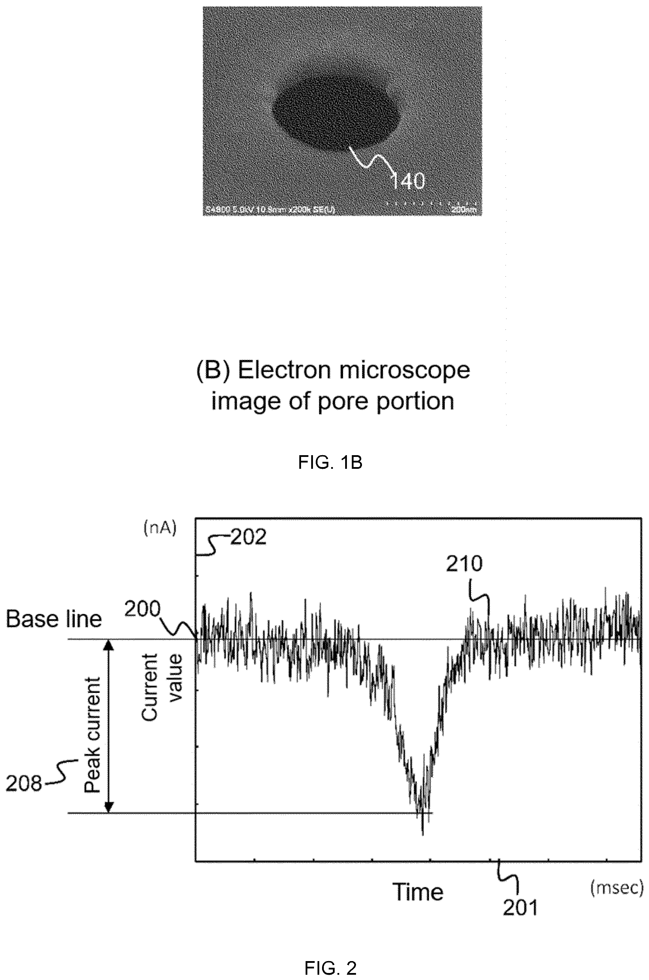

In the present invention, antibody-modified particles, the surface of which is modified with an antibody that specifically binds to the protein to be detected or quantified (hereinafter referred to as the "target antigen" or simply "antigen"), are measured for their agglutination state in which the particles bind via the target antigen using a sensor such as that shown in FIG. 1, rather than using conventional optical methods.

FIG. 1A shows an example of the device structure of a sensor used in the present invention. The sensor 100 has a cross-sectional structure in which two chambers 110 and 120 are separated by a partition wall 141 and connected via a pore 140 provided in the partition wall 141. Electrodes 112 and 122 are provided in the two chambers, respectively. A sample containing particles suspended in an electrolyte is introduced into chamber 110 through inlet 111, and the electrolyte is introduced into chamber 120 through inlet 121, and a voltage is applied to the two electrodes by voltage source 152. For example, when a voltage is applied between electrodes 112 and 122, an ionic current flows through the pore. In addition, as used herein, the term "chamber" refers to a portion capable of storing a sample solution (electrolyte solution). In addition, as used herein, an electrode is defined as being "inside the chamber" if it is in a position where it can come into contact with the sample liquid in the chamber (that is, where electricity can be passed therethrough). In addition, as used herein, "filling" the chamber does not necessarily mean filling the entire volume of the chamber, but may leave some void as long as the sensor functions. That is, as used herein, "filling" may be considered interchangeably with "injection" or "introduction." In addition, as used herein, when the sample itself is a liquid, it may be considered as a kind of electrolyte. FIG. 1B shows an electron microscope image of a pore 140 used to measure the pulse waveform in the following experiment. The diameter of the pore is selected to be larger than the diameter of the antibody-modified particle to be measured.

As shown in FIG. 1A, when a particle present in chamber 110 passes through aperture 140, the ionic current is temporarily interrupted and returns to normal after the particle passes through chamber 120. Therefore, every time a particle passes through the pore 140, the ion current flowing between the electrodes in FIG. 1 exhibits a pulse-like transient change as shown in FIG. 2. In the example of Fig. 1, this is measured by ammeter 151. In the example shown in FIG. 2, the pulse signal is a current value 202 at each time 201. In another embodiment, the vertical axis 202 may be a voltage value. The baseline is the current value when there is no pulse (or the average over a certain period of time if there is noise), and the peak current 208 is the difference between the baseline and the current value at which the largest current drop occurs in the pulse waveform (or the average over a certain period of time if there is noise).

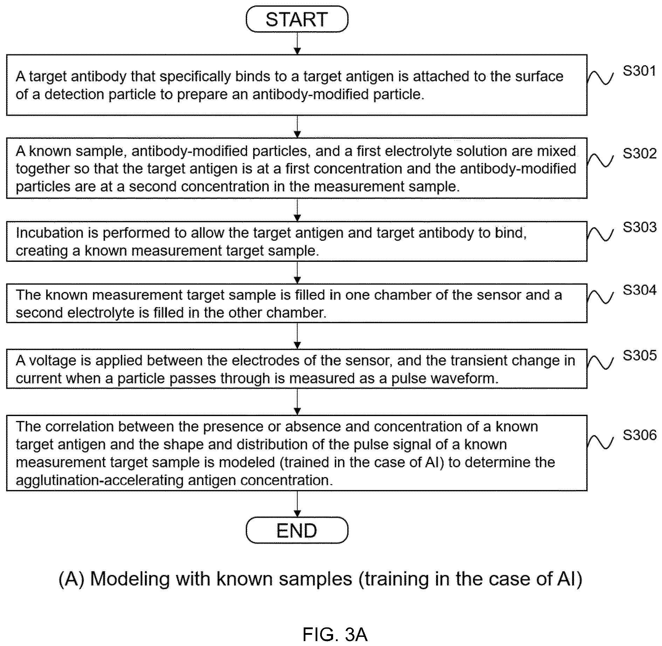

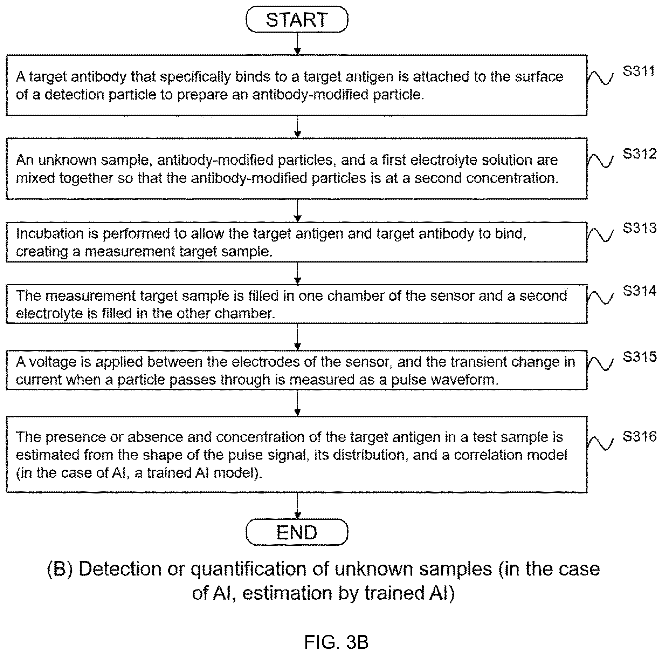

FIG. 3 outlines the principle and procedure for detecting or quantifying an antigen in a specimen according to the present invention. In the present invention, before measuring an unknown sample, a known sample in which the presence or absence and concentration of a target antigen are known is measured as shown in FIG. 3A, and the correlation between the presence or absence and concentration of the target antigen and the shape or distribution characteristic of the pulse signal obtained by the method of the present invention is modeled. Next, as shown in FIG. 3B, an unknown sample in which the presence or absence and concentration of the target antigen are unknown is measured and compared with the model to detect and quantify the target antigen in the unknown sample. In a preferred embodiment of the present invention, the sensor used to measure a known measurement sample as shown in FIG. 3A and the sensor used to measure an unknown measurement sample as shown in FIG. 3B may be physically separate sensors (For example, the sensors may be of the same product type but physically separate, or sensors of different product types may be used separately). By doing this, it becomes possible to preferably implement the present invention even if, for example, the location/person where modeling is performed using a known measurement sample and the location/person where measurement of an unknown measurement sample is performed using that model (data) are located far apart. Alternatively, in another embodiment, the same physical sensor in reality may be used to measure the known and unknown measurement samples. In addition, as used herein, a known sample containing a target antigen of a known antigen concentration and antibody-modified particles whose surface is modified with an antibody that specifically binds to the target antigen may be referred to as a "known measurement target sample." In addition, a sample containing an unknown sample containing a target antigen with an unknown antigen concentration and antibody-modified particles whose surfaces are modified with antibodies that specifically bind to the target antigen may be referred to as an "unknown measurement target sample." These measurement target samples can be mixed and prepared depending on the reaction efficiency of the target antigen and antibody. These measurement target samples may be interpreted as including an electrolyte for electrically connecting the chambers. The electrolytes injected into both chambers may have different compositions or may have the same composition. Any electrolyte known in the art may be used depending on the application of the present invention.

In the method shown in FIG. 3A, first, an antibody (hereinafter referred to as a target antibody) that specifically binds to an antigen to be detected or quantified (hereinafter referred to as a target antigen) is modified onto a detection particle to prepare an antibody-modified particle (step S301). Next, the antibody-modified particles, a first electrolyte solution, and a sample in which the presence or absence and concentration of a target antigen are known are mixed (step S302). The mixed sample contains the target antigen at a first concentration and the antibody-modified particles at a second concentration. In the present invention, the second concentration is set according to the antigen concentration of the unknown sample that is to be detected or quantified in practice, according to the conditions described below. Next, incubation is performed so that the target antigen, if present in the known sample, binds to the antibody-modified particles, to prepare a known measurement target sample (step S303). The known measurement target sample contains a first concentration of the target antigen and a second concentration of the antibody-modified particles. Next, one chamber of the sensor 100 is filled with the known measurement target sample, and the other chamber is filled with a second electrolyte solution, thereby establishing electrical continuity between the electrodes 112 and 122 (step S304). Thereafter, a voltage is applied between the electrodes of the sensor, and a pulse waveform group consisting of a plurality of pulse waveforms measured at a known pulse frequency, which are generated as the particle passes through the pore 140, is measured (step S305). Then, a correlation is modeled between the presence or absence and concentration of the known target antigen and the pulse features expressing the shapes of the individual pulse waveforms obtained in step S305 above, or the distribution of the pulse feature within the pulse waveform group. This modeling may be done by analytical methods or by training an Al model. The Al model is trained using the pulse waveform itself, pulse features that express the characteristics of the pulse waveform, and distribution features that express the distribution characteristics of the pulse waveform features as a teacher data, and also using the presence or absence and concentration of a known target antigen as a teacher label. When using an Al model, any algorithm may be used, such as a support vector machine, a linear discriminant transform, a k-nearest neighbor method, a decision tree, or an ensemble learning of these, or various deep learning and recursive algorithms. When determining whether or not a target antigen is contained in an unknown sample using the method shown in FIG. 3B, a classification algorithm or the like that outputs presence or absence is used. When quantifying the target antigen using the method of FIG. 3B, a regression algorithm or the like that outputs a continuous amount may be used.

After the above modeling (or Al training), the measurement results of an unknown sample, in which the presence or absence and concentration of the target antigen are unknown, are compared with the model (or trained AI) to estimate the presence or absence and concentration of the target antigen, as illustrated in FIG. 3B. The processes in steps S311 to S315 are generally similar to those in FIG. 3A, but in the sample mixing in step S312, since the concentration of the target antigen is unknown, the antibody-modified particles are set to the same second concentration as in step S302. In addition, in step S315, a pulse waveform group consisting of a plurality of pulse waveforms measured at an unknown pulse frequency is measured. Steps S301 and S311 use the same antibody and the same detection particles, and prepare antibody-modified particles according to the same protocol.

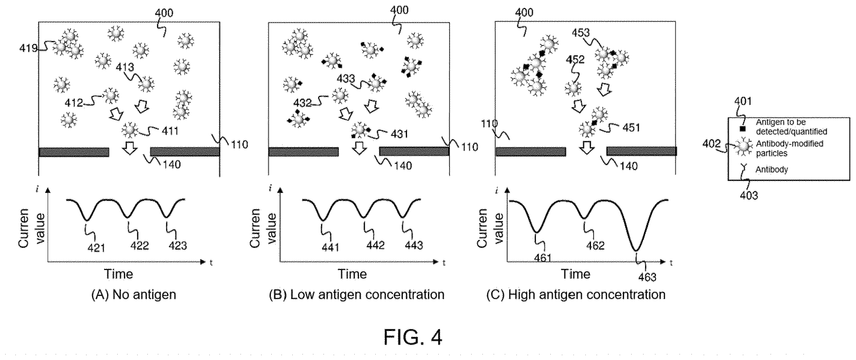

Next, the principle of detection or quantification of a target antigen in the present invention will be described. FIG. 4A to 4C are schematic diagrams showing a state in which the chamber 110 is filled with a measurement target sample. When the target antigen is not contained in the known sample or the unknown sample, the target antigen bound to the target antibody is not present in the measurement sample as shown in FIG. 4A. When a voltage is applied between the electrodes 112 and 122 in this state, the antibody-modified particles in the measurement sample pass through the pore 140 alone, as shown in FIG. 4A. In the example of FIG. 4A, the pulse signals generated when antibody-modified particles 411, 412 and 413 pass through the pore 140 are 421, 422 and 423, respectively. If the measurement target sample does not contain the target antigen, the antibody-modified particles will not bind via the target antigen. For this reason, most antibody-modified particles pass through the pores alone, but some, such as particle 419, may nonspecifically bind without the target antigen, and antibody-modified particles may be observed passing through the pores in a bound state.

When a target antigen is present in the measurement sample, the target antigen is loaded into chamber 110 in a state in which it is bound to antibody-modified particles, as shown in FIG. 4B and FIG. 4C. FIG. 4B shows the case where the antigen is dilute, and the target antigen is bound to the antibody-modified particle. However, as shown in FIG. 4C where the antigen concentration is high, there is almost no aggregation between the antibody-modified particles mediated by the target antigen.

As shown in FIG. 4C, when the concentration of the target antigen is high, the antibody-modified particles form aggregates via the target antigen. The waveform of the pulse signal generated when these pass through the pore has a peak current value greater than that of the pulse signal generated when the antibody-modified particle passes through the pore alone. This is because when a large particle passes through a pore, the ionic current through the pore is impeded to a greater extent than when a small particle passes through the pore. For example, in FIG. 4C, the pulse signals of agglomerate 451, particle 452 and agglomerate 453 passing through pore 140 are 461, 462 and 463, respectively. This is the reason why the peak current value of the pulse signal is greater for agglomerate 451 than for single particle 452, and is further greater for agglomerate 453.

As an example for verifying the principle, the present inventors conducted experiments and analyses using the method according to the present invention, using PSA (Prostate Specific Antigen) as the target antigen and antibody-modified particles attached to latex detection particles by hydrophobic interactions.

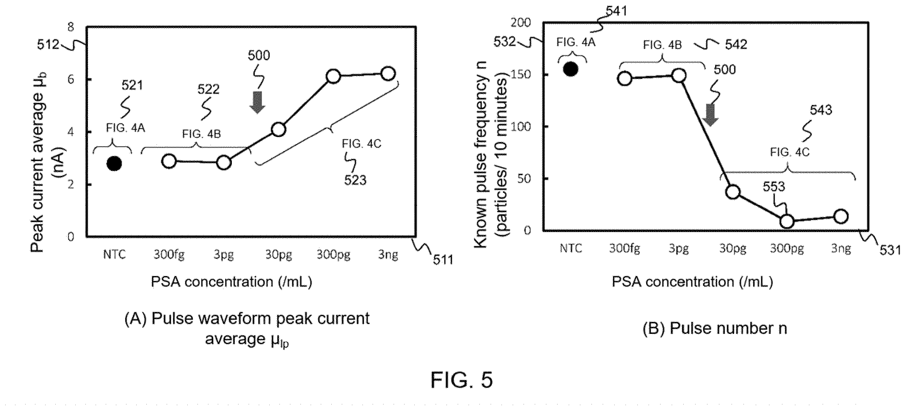

FIG. 5 illustrates the distribution of pulse waveform characteristics, which are the results of measuring known samples for the presence/absence and concentration of PSA, which is an example of a target antigen. FIG. 5A shows the average peak current 512 of the pulse waveforms in the pulse waveform group for each measurement for each target antigen concentration 511. NTC 521 represents the result for a measurement sample that does not contain the target antigen. In the measurement sample not containing the target antigen, the number of pulses per measurement was 541. On the other hand, when the target antigen concentration was 30 pg/mL or 300 pg/mL, aggregates were formed as shown in FIG. 3C, and the average peak current value increased 523 as shown in FIG. 5A. In addition, at this target antigen concentration, many aggregates are formed, resulting in the consumption of individual antibody-modified particles, and the number of pulses per measurement decreases 543 as shown in FIG. 5B.

From these results, it is estimated that in the case of measurement samples prepared using the protocol of the present invention in steps S301 to S303 with various target antigen PSA concentrations, when the PSA concentration is approximately 10 pg/mL or less, the state is as shown in FIG. 4B, and when the PSA concentration is 10 pg/mL or more, the state is as shown in FIG. 4C.

Taking into account the results of experiments on other target antigens equivalent to those in FIG. 5, the inventors have found that when the target antigen concentration is successively increased from the state in which there is no target antigen in FIG. 4A to fg/mL and then to pg/mL, the number of pulse waveforms (for example, 461 and 463) resulting from aggregation in FIG. 4C does not generally increase gradually. In other words, until a certain target antigen concentration reaches a threshold value, as shown by pulse waveforms 441 to 443 in FIG. 4B, the peak current of the pulse waveform remains almost unchanged compared to when there is no target antigen. In this case, it is believed that the amount of target antigen attached to the antibody-modified particles gradually increases depending on the concentration of the target antigen. On the other hand, when the concentration of the target antigen exceeds the threshold value, aggregation of the antibody-modified particles via the target antigen proceeds rapidly as shown in FIG. 4C. In the above-mentioned experiment using antibody-modified particles whose surfaces were modified with anti-PSA antibodies, which the authors used in step S301, this threshold was about 10 pg/mL. The present inventors conducted experiments on various types of proteins (antigens), and found that the threshold for the states shown in FIG. 4B and FIG. 4C varies greatly, from the order of pg/mL to the order of µg/mL, depending on the combination of the target antigen, antibody-modified particles, and their respective concentrations. On the other hand, the phenomenon of rapid progression of agglutination once a certain antigen concentration threshold has been reached was common. This threshold is referred to herein as the agglutination-accelerating antigen concentration. In addition, in another embodiment, a similarly definable antibody concentration threshold is referred to as the agglutination-accelerating antibody concentration. Furthermore, in this specification, the term "agglutination accelerating concentration" may also be used as a comprehensive concept of agglutination accelerating antigen concentration and agglutination accelerating antibody concentration.

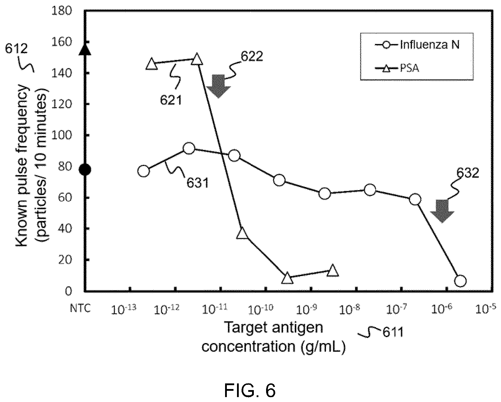

FIG. 6 shows an example of target antigen concentrations 611 with known pulse frequencies 612 for two types of target antigens. In the concentration range shown in FIG. 4C, rapid aggregation occurs, resulting in consumption of the antibody-modified particles and a significant decrease in the number of pulses. In the case of measurement results 631 of recombinant N protein of influenza A virus, the agglutination accelerating antigen concentration 632 can be determined to be on the order of approximately 1 ng/mL. In contrast, according to the PSA measurement result 621, the agglutination accelerating antigen concentration is determined to be 10 pg/mL.

Returning to FIG. 5, the behavior of the pulse waveform distribution in the concentration range equal to or higher than the agglutination-accelerating antigen concentration will be described using the results of PSA as an example. In FIG. 5A, the peak current average µb 512, which is an example of a distribution feature described later, is clearly larger in a concentration region 523 higher than the agglutination accelerating antigen concentration 500 than NTC 521, which does not contain the target antigen, and has a strong positive correlation with the concentration of the target antigen, PSA. Similarly, in FIG. 5B, the known pulse number frequency 532, which is another example of a distribution feature described later, is clearly smaller in a concentration region 543 higher than the agglutination accelerating antigen concentration 500 than NTC 541, which does not contain the target antigen, and has a strong negative correlation with the concentration of PSA, which is the target antigen. By using these results together, it is possible to model the relationship between the presence or absence and concentration of a target antigen in a known sample and the shape and distribution of a pulse signal in a concentration range of 500 or more of an agglutination accelerating antigen concentration (step S306). Therefore, in one example of FIG. 5 according to the present invention, this correlation can provide a means for detecting or quantifying PSA with a detection limit of 10 pg/mL. That is, as used herein, "detection" may be considered to be a type of "quantification."

In detection, the agglutination accelerating antigen concentration is calculated based on the pulse waveform shape and distribution characteristics from the data such as that shown in FIG. 5 (step S306), and then in the detection of the target antigen in the unknown sample shown in FIG. 3B (step S316), the presence or absence of the antigen in the unknown sample can be estimated by comparing it with the threshold value. In addition, in quantification, for example, a correlation coefficient between the target antigen and the shape and distribution characteristics of the pulse waveform is calculated from data such as that shown in FIG. 5 (step S306), and in detection of the target antigen in the unknown sample shown in FIG. 3B (step S316), an estimated antigen concentration in an unknown sample can be calculated based on the correlation coefficient.

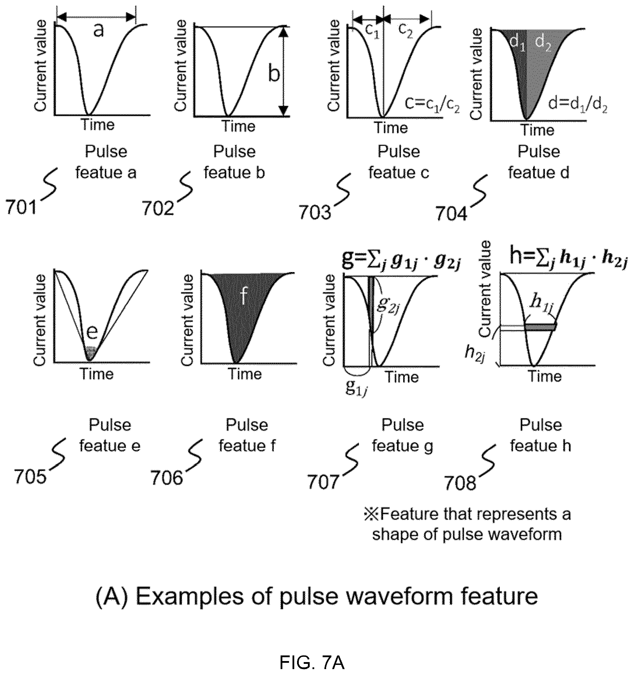

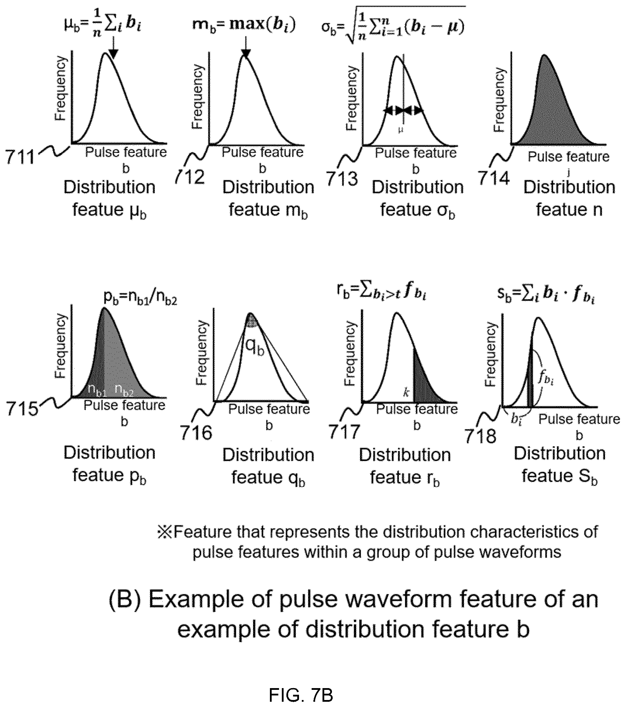

FIG. 5 shows that the detection or quantification of a target antigen can be performed at a target antigen concentration equal to or higher than the agglutination acceleration region by utilizing the distribution characteristics of the pulse waveform, that is, the peak current average µ_{b} and the pulse number n. This is just one example, and the present invention may utilize any pulse waveform characteristics or distribution characteristics. FIG. 7A shows an example of a feature that expresses the shape of one pulse waveform, and FIG. 7B shows an example of a feature that expresses the characteristics of the shape of a histogram for the frequency distribution of one feature in a pulse waveform group obtained from one measurement. The former is called a pulse waveform feature and the latter is called a distribution feature. The pulse waveform peak current average µ_{b} 512 shown in FIG. 5 corresponds to the distribution feature µ_{b} obtained by averaging the pulse waveform feature, that is, the peak current value b in FIG. 7A, for n pulses in one pulse waveform group. The number of pulses n shown in FIG. 5 is the number of pulses n in one pulse waveform group. It should be noted that the vertical axis of FIG. 7A is the current value, and the vertical axis of FIG. 7B is the frequency. The present invention may use any number of pulse waveform features and distribution features, and may also use any combination of pulse waveform features and distribution features. If the number of types of pulse waveform features is N and the number of types of distribution features is M, then the number of types of distribution features that can be used in the present invention is M × N.

In addition, for detection purposes, it is also possible to create an Al detector that estimates the presence or absence of a target antigen by training a binary classification Al using not only the average peak current of the pulse waveform and the number of pulses, which are examples of feature shown in FIG. 5, but also various pulse waveform features and their distribution features as training data and the presence/absence of the target antigen as training labels (step S306). Furthermore, in addition to the features shown in FIG. 5, such as the average peak current and the number of pulses, various pulse waveform features and their distribution features can be used as training data, and the concentration of the target antigen can be used as a training label to train a regression Al or a multi-value classification Al, thereby creating an Al quantifier that estimates the presence or absence of a target antigen (step S306). Then, for a sample in which the presence or absence and concentration of a target antigen are unknown, the presence or absence and concentration of the target antigen can be estimated by the procedure shown in FIG. 3B. The PSA results shown in FIG. 5 are only an example, and the above-described method of the present invention can be applied to any antigen and any antigen for which an antibody that specifically binds to the antigen exists.

Next, the pre-measurement processes in steps S301 to S303 and steps S311 to S313 in FIG. 3 will be described. Hereinafter, these processes are collectively referred to as "pre-measurement processing." There are two preferable requirements for the pre-measurement processing: the first requirement is appropriate control of the agglutination accelerating antigen concentration according to the application, and the second requirement is realization of stable measurement.

The first requirement for pre-measurement processing will be described below. In clinical applications, the concentration range of the target antigen to be detected or quantified varies greatly depending on the target antigen. For example, in the description of FIG. 5, the normal value of PSA, which is given as an example of a target antigen, in a prostate cancer test is set to 4 ng/mL or less. On the other hand, the normal blood concentration of CRP (C-Reactive Protein), a representative inflammatory marker, is 3 µg/mL or less. For this reason, for example, when a testing means for detecting or quantifying target antigens using these proteins as target antigens utilizes the state shown in FIG. 4C, it is preferable to appropriately control the agglutination-accelerating antigen concentration in accordance with clinical requirements.

The second requirement is to realize stable passage of the antibody-modified particles or aggregates thereof through the pores. Even if pre-measurement processing is performed to realize state changes as shown in the upper diagrams of FIG. 4A and FIG. 4C in accordance with the amount of target antigen, it is not necessarily possible to stably detect pulse waveforms as shown in the lower diagrams of the respective figures. For example, it is preferable to be able to acquire a sufficient number of pulses to analyze the characteristics of the pulse waveform or the characteristics of its distribution, as shown in FIG. 5 or FIG. 6, within a practical measurement time. In the example shown in FIG. 5B, when the PSA concentration 553, which is the target antigen, is 300 pg/mL, the number of pulses is extremely small compared to NTC. In such cases, it is preferable to adjust the concentration of the antibody-modified particles mixed with the sample in order to optimize the length of measurement time to obtain a sufficient number of pulse waveforms for S306 and S316 in the target antigen concentration range where the PSA concentration is 553 or higher.

In order to satisfy these first and second requirements, it is preferable to optimize the pre-measurement processing in steps S301 to S303 and S311 to S313 for each type of target antigen and each clinical requirement. In the following, an example of an optimal concentration of antibody-modified particles to be mixed with the sample in steps S302 and S312 will be described, which is one of the pre-measurement treatment methods that can satisfy the above first and second requirements.

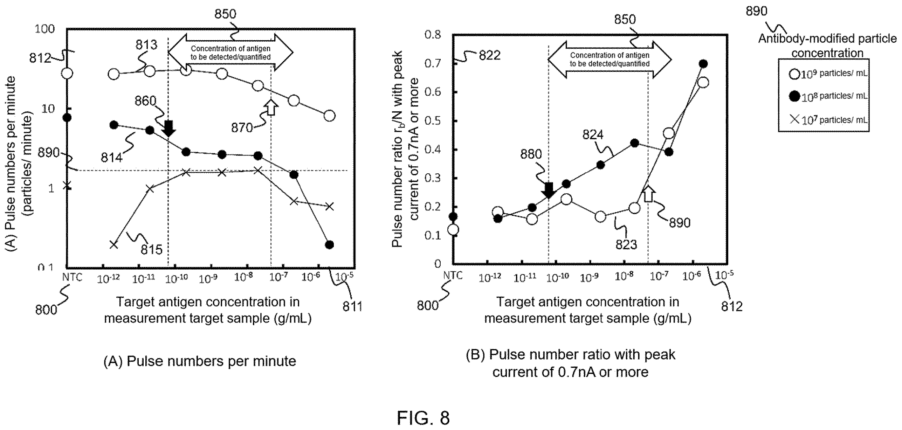

Further, as an example different from that shown in FIG. 5, a method will be described in which influenza N protein is used as the target antigen, antibody-modified particles in which anti-influenza N protein antibodies are modified as measurement particles, and pre-measurement processing is performed based on the results of measuring the pulse waveform using a method according to the present invention. FIG. 8 is a plot of the number of pulses per minute and the ratio of the number of pulses equal to or greater than the lower limit of peak current versus the target antigen concentration of influenza N protein. Here, the target antigen concentrations in the known measurement samples were adjusted to 0 g/mL (NTC), 2 pg/mL to 2 µg/mL in 10-fold increments. The concentrations are shown on the horizontal axes 811 and 812 of FIG. 8. FIG. 8 shows the results of measurements performed by the method of the present invention using antibody-modified particles at three concentrations (the "second concentration" referred to in the above Aspect 2) of 7 × 10⁹ particles/mL, 7 × 10⁸ particles/mL, and 7 × 10⁷ particles/mL, for each target antigen concentration. The vertical axis 812 in FIG. 8A represents the average number of pulses per minute for the pulse waveform group obtained in one measurement, and the vertical axis 822 in FIG. 8B represents the pulse number ratio r/n (t>0.7 nA) of the peak current value of 0.7 nA or more within one measured pulse waveform group. This is obtained by dividing the distribution feature amount r 717 by the number of pulses n 714 shown in FIG. 7. As described above, as the aggregation of the antibody-modified particles progresses, the peak current value increases, and therefore this r /n is considered to be a distribution feature amount reflecting the aggregate mass.

In the example shown in FIG. 8, when the antibody-modified particle concentration (the "second concentration" in the above-mentioned Aspect 2) is 10⁹ particles/mL, the target antigen concentration in the measurement sample is approximately 10⁻⁷ g/mL or higher, and (a) the distribution feature amount n significantly decreases 870, and (b) the ratio of the distribution feature amount r/n significantly increases 890. In this case, it is considered that at a target antigen concentration of 10⁻⁷ g/mL or more, aggregation between the antibody-modified particles proceeds rapidly, resulting in the state shown in FIG. 4C. That is, in this example, this target antigen concentration threshold can be defined as the aggregation-accelerating antigen concentration. In addition, in the example of FIG. 7, when the antibody-modified particle concentration is 10⁹ particles/mL, the agglutination-accelerating antigen concentration can be about 10⁻⁷ g/mL. Similarly, when measurement pretreatment is performed with an antibody-modified particle concentration of 10⁸ particles/mL, when the target antigen concentration in the measurement sample is approximately 10⁻¹⁰ g/mL or higher, (a) the number of pulses decreases significantly 860, and (b) the ratio of the number of pulses with a peak current of 0.7 nA or higher increases significantly 880. In addition, it should be noted that the vertical axis in FIG. 8A is expressed logarithmically.

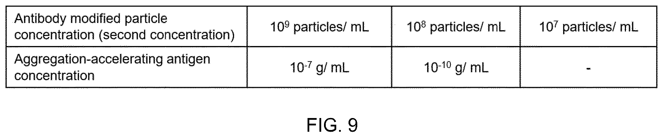

The relationship between the antibody-modified particle concentration (the "second concentration" in the above Aspect 2) and the agglutination-accelerating antigen concentration in the example of FIG. 8 is summarized in FIG. 9. When the antibody-modified particle concentration is 10⁹ particles/mL, the agglutination-accelerating antigen concentration can be approximately 10⁻⁷ g/mL, and when the antibody-modified particle concentration is 10⁸ particles/mL, the agglutination-accelerating antigen concentration can be approximately 10⁻¹⁰ g/mL. For example, when detecting and quantifying influenza N protein as a target antigen for an unknown sample in concentration region 850 in FIG. 8, if the antibody-modified particle concentration is 10⁹ particles/mL, distribution features 813 and 823 in FIG. 8 will be used. However, in the detection/quantification target antigen region 850, these distribution feature amounts are not significantly different from NTC 800, and the correlation with the target antigen concentration is not clear. However, if the antibody-modified particle concentration is set to 10⁸ particles/mL, the distribution features 814 and 824 in FIG. 8 can be used, enabling more accurate detection and quantification. That is, in the present invention, by setting the antibody-modified particle concentration so that the target antigen concentration to be detected/quantified is higher than the agglutination-accelerating antigen concentration, it becomes possible to detect or quantify the target antigen with high accuracy. This is a method that meets the first requirement for pre-measurement processing.

The agglutination accelerating antigen concentration in the present invention can be determined by determining the dependency of the pulse waveform feature amount or distribution feature amount on the target antigen concentration in the measurement sample, as exemplified in FIG. 5, FIG. 6, or FIG. 8. The target antigen concentration at which the pulse waveform feature value or distribution feature value starts to change more significantly than in the low concentration range in response to changes in the target antigen concentration of a known sample (in other words, the target antigen concentration at which the change in the pulse waveform feature value or distribution feature value exceeds a predetermined threshold value.) may be determined as the agglutination accelerating antigen concentration. The amount of change can be measured, for example, by differentiating the correlation shown in FIG. 5, 6, or 8 with respect to the concentration (horizontal axis) (by determining the slope of the tangent to the approximation curve of the plot shown in the figure).

Further, in some embodiments, a threshold for the amount of change may be determined as follows: For example, referring to FIG. 5A, the change in the peak current average µ_{b} when the target antigen concentration increases 10-fold from 300 fg/mL to 3 pg/mL is approximately a 7% increase. On the other hand, the change in µ_{b} from 3 pg/mL to 30 pg/mL increases by about 36%. Based on this, 500 near 10 pg/mL may be taken as the agglutination-accelerating antigen concentration. Similarly, in FIG. 5B, the change in µ_{b} from 3 pg/mL to 30 pg/mL is greater than the change in pulse number n from 300 fg/mL to 3 pg/mL. For example, when the difference between a distribution feature at an agglutination accelerating antigen concentration and the same distribution feature at an antigen concentration 10 times that concentration is defined as a first difference, and the difference between the distribution feature at an agglutination accelerating antigen concentration and the same distribution feature at an antigen concentration 1/10 of that concentration is defined as a second difference, the agglutination accelerating antigen concentration may be determined so that the first difference is at least twice the second difference. In addition, the above is merely one example, and in another embodiment of the present invention, any method may be used as long as it determines the agglutination acceleration concentration by utilizing a change in a pulse waveform feature or distribution feature with respect to the target antigen concentration, and the agglutination acceleration concentration may be determined by using a single pulse waveform feature or distribution feature, or by combining multiple pulse waveform feature or distribution feature.

For detection or quantification of target antigens in even more sparse areas, the concentration of antibody-modified particles may be further reduced. However, the number of pulses measured per unit time generally has a strong positive correlation with the particle concentration of the antibody-modified particles. This is because the number of particles passing through the pore is roughly proportional to the particle concentration in the sample. For example, in FIG. 8A, when the antibody-modified particle concentration is 10⁷ particles/mL, the pulse frequency is 2 particles or less per minute regardless of the target antigen concentration.

In a sensor such as that shown in FIG. 1, the pulse waveform measured generally has statistical variations. Furthermore, for example, the shape of the pulse waveform will be different when a particle passes through the center of a circular aperture and when it passes through a position offset from the center. It is unavoidable that there will be some variation in the particle size and dimensions of the detection particles used to prepare the antibody-modified particles. Furthermore, in the region above the agglutination-accelerating antigen concentration, the agglutination of antibody-modified particles is not uniform, but is in a state in which large aggregates formed by the aggregation of many particles and small aggregates formed by the aggregation of only a few particles are widely distributed. For this reason, in a preferred embodiment, in both the creation of the correlation model in FIG. 3A and the detection or quantification of the target antigen from an unknown sample in FIG. 3B, by measuring at least about 100 pulse waveform groups per measurement, the effects of these statistical variations can be reduced in steps S306 and S316.

In the example of FIG. 8A, when the antibody-modified particle concentration is 10⁷ particles/mL, the pulse frequency is 2 particles/min regardless of the target antigen concentration, so that measuring 50 pulse waveforms requires approximately 40 minutes of measurement. If an antibody-modified particle concentration of 10⁶ particles/mL is used, the measurement time will be 400 minutes. In order to obtain the effect of rapidity, it is preferable that the pulse waveforms can be acquired at a frequency of at least about 2 pulses per minute in the measurements of steps S305 and S315. This is a method that meets the second requirement regarding the above-mentioned pre-measurement processing.

In summary, as a preferred sample pretreatment, by selecting an antibody-modified particle concentration such that, first, the agglutination-accelerating antigen concentration is lower than the antigen concentration to be detected or quantified, and second, the pulse waveform frequency is 2 particles/minute or more, a means for detecting or quantifying a target antigen with higher accuracy and speed can be provided.

The method according to the present invention as described above can be implemented by a computer device or terminal having a processor (which may be a single processor or a multi-core processor, and may also include a microprocessor). Such a computing device may be a device integrated with a sensor, such as sensor 100 of FIG. 1. Alternatively, it may be a computer device that is operatively connected to such a sensor via a network (whether wired or wireless, such as the Internet, a dedicated closed network, or a LAN).

The computer device that can be used in relation to the present invention may take any form, such as a workstation, a tablet, a smartphone, and the like.

In one embodiment of the present invention, a computer-readable program and a medium for storing the program can be provided, and the program can be executed by a processor to perform any of the steps included in the above-described method.

## Claims

1. A method for quantifying an antigen or antibody, the method using a sensor comprising two chambers separated by a partition wall and communicating with each other through a pore, and an electrode in each of the two chambers; the method comprising:
preparing a known measurement target sample by mixing:
antibody-modified particles having an antibody that specifically binds to the antigen attached to a particle surface, or antigen-modified particles having an antigen that specifically binds to the antibody attached to a particle surface,
a first electrolyte solution, and
a known sample having a known concentration of the antigen or the antibody,
wherein the antigen or the antibody is contained at a first concentration, and the antibody-modified particles or the antigen-modified particles are contained at a second concentration, respectively;
filling one of two chambers of a first sensor with the known measurement target sample;
filling the other of the two chambers of the first sensor with a second electrolyte solution to electrically connect the two chambers through a pore;
applying a voltage between the electrodes of each of the two chambers and causing an ion current to flow between the electrodes via the pore, and measuring transient change in the ion current as a known pulse waveform group consisting of a plurality of known pulse waveforms measured at a known pulse frequency equal to or higher than a lower limit frequency;
creating a correlation model between a known pulse waveform feature expressing a shape of the known pulse waveform, or a known distribution feature expressing a distribution characteristic of the known pulse waveform feature within the group of known pulse waveforms, and a concentration of the antigen or the antibody in the known sample;
using the correlation model, determining a value at which a change in the concentration of the antigen or the antibody with respect to the known pulse waveform feature or the known distribution feature exceeds a predetermined threshold, and defining the value as an agglutination accelerating concentration; and
estimating a concentration of the antigen or the antibody contained in an unknown sample by using the correlation model and the agglutination accelerating concentration.

2. The method according to claim 1, wherein the step of estimating the concentration of the antigen or the antibody contained in the unknown sample by using the correlation model and the agglutination accelerating concentration comprises:
preparing an unknown measurement target sample by mixing:
the antibody-modified particles or the antigen-modified particles,
the first electrolyte solution, and
the unknown sample,
wherein the antibody-modified particles or the antigen-modified particles are contained at a second concentration;
filling the unknown measurement target sample into one of the two chambers of a second sensor;
filling the other of the two chambers of the second sensor with the second electrolyte solution to electrically connect the two chambers through a pore;
applying a voltage between the electrodes of each of the two chambers and causing an ion current to flow between the electrodes via the pore, and measuring transient change in the ion current as an unknown pulse waveform group consisting of a plurality of unknown pulse waveforms measured at an unknown pulse frequency equal to or higher than a lower limit frequency; and
estimating a concentration of an antigen or antibody in the unknown sample by comparing an unknown pulse waveform feature that expresses a shape of the unknown pulse waveform, or an unknown distribution feature that expresses a distribution characteristic of the unknown pulse waveform feature within the unknown pulse waveform group, with the correlation model.

3. The method according to claim 1 or 2, wherein the second concentration is set so that the aggregation accelerating concentration is lower than the first concentration.

4. The method according to claim 2, wherein the lower frequency limit of the unknown pulse rate is 2 pulses per minute.

5. The method according to claim 1 or 2, wherein the aggregation accelerating concentration is determined such that:
a difference between a reference pulse waveform feature at the agglutination accelerating concentration and a reference pulse waveform feature at an antigen concentration or antibody concentration that is 10 times the agglutination accelerating concentration is equal to or greater than twice of
a difference between the reference pulse waveform feature at the agglutination accelerating concentration and the reference pulse waveform feature at an antigen concentration or antibody concentration that is 1/10 of the agglutination accelerating concentration.

6. The method according to claim 1 or 2, wherein the aggregation accelerating concentration is determined such that:
a first difference between a distribution feature at the agglutination accelerating concentration and a reference distribution feature at an antigen concentration or antibody concentration that is 10 times the agglutination accelerating concentration is equal to or greater than twice of
a second difference between the distribution feature at the agglutination accelerating concentration and the reference distribution feature at an antigen concentration or antibody concentration that is 1/10 of the agglutination accelerating concentration.

7. The method according to claim 2, wherein the first electrolyte and the second electrolyte have different compositions.

8. The method according to claim 2, wherein the first sensor and the second sensor are the same sensor in reality.

9. A program, comprising computer-readable instructions configured to be executed by a processor in a computer in which the processor is configured to be connected to a sensor via a network, the sensor comprising two chambers separated by a partition wall and communicating with each other through a pore, and an electrode in each of the two chambers; the computer readable instructions configured to execute the processor to perform the steps of:
a step of preparing a known measurement target sample by mixing:
antibody-modified particles having an antibody that specifically binds to an antigen attached to a particle surface, or antigen-modified particles having an antigen that specifically binds to an antibody attached to a particle surface,
a first electrolyte solution, and
a known sample having a known concentration of the antigen or the antibody,
wherein the antigen or the antibody is contained at a first concentration, and the antibody-modified particles or the antigen-modified particles are contained at a second concentration, respectively;
a step of filling one of two chambers of a first sensor with the known measurement target sample, by the processor;
a step of filling the other of the two chambers of the first sensor with a second electrolyte solution to electrically connect the two chambers through a pore, by the processor;
a step of applying a voltage between the electrodes of each of the two chambers and causing an ion current to flow between the electrodes via the pore, and measuring transient change in the ion current as a known pulse waveform group consisting of a plurality of known pulse waveforms measured at a known pulse frequency equal to or higher than a lower limit frequency, by the processor;
a step of creating a correlation model between a known pulse waveform feature expressing a shape of the known pulse waveform, or a known distribution feature expressing a distribution characteristic of the known pulse waveform feature within the group of known pulse waveforms, and a concentration of the antigen or the antibody in the known sample, by the processor;
a step of using the correlation model, determining a value at which a change in the concentration of the antigen or the antibody with respect to the known pulse waveform feature or the known distribution feature exceeds a predetermined threshold, and defining the value as an agglutination accelerating concentration, by the processor; and
a step of estimating a concentration of the antigen or the antibody contained in an unknown sample by using the correlation model and the agglutination accelerating concentration, by the processor.
